# EUROPEAN PATENT APPLICATION

(11) **EP 1 782 812 A1**
(43) Date of publication of application: **09.05.2007**
(21) Application number: 05762969.3
(22) Date of filing: 23.06.2005
(51) Int. Cl.: A61K 31/549, A61P 25/00

(54) **COMPOUNDS FOR THE TREATMENT OF INFLAMMATION OF THE CENTRAL NERVOUS SYSTEM**

(30) Priority: 23.06.2004 ES 200401627
(71) Applicant: Neurotec Pharma, S.L., 08028 Barcelona (ES)
(72) Inventor: MAHY GÈHENNE, Josette-Nicole, E-08328 Alella (ES); RODRÍGUEZ ALLUÉ , Manuel José, E-08980 Sant Feliu de Llobregat (ES); PUGLIESE, Marco, E-08810 Sant Pere de Ribes (ES)
(86) International application number: PCT/ES2005/000356
(87) International publication number: WO 2006/000607

(57) **Abstract**

K_{ATP} channel openers (KCOs) are useful for the prophylactic and/or therapeutic treatment of CNS chronic inflammation associated with a disease or state in a mammal, including a human. The administration of KCOs, including the groups of benzopirans, cyanoguanidines, thioformamides, benzothiadiazines, pyridyl nitrates, pyrimidine sulfates, cyclobutenediones, DHP-related compounds, tertiary carbinols, 6-sulfonil-chromenes, 1,2,3-triazoles, pyridothiadiazines, benzothiazines, halogenquinazolins and phenylbenzimidazoles, and in particular, the compound diazoxide, result in a reduction of reactive microglial response in various CNS pathologies such as axonal injury, brain tumors, traumatic damage, neurodegeneration, spinal cord injury, infectious and autoimmune diseases. KCOs, isotopically modified, are also useful for the preparation of diagnostic agents for detection and follow-up of CNS chronic inflammation.

## Description

This invention relates to the field of human and animal medicine, and specifically to compounds for the treatment and diagnosis of diseases, in particular, diseases related with the central nervous system inflammation.

### BACKGROUND ART

Microglia are distributed in non-overlapping territories throughout the central nervous system (CNS). In functional terms, microglia represents the network of immune accessory cells throughout the brain, spinal cord and neuroocular structures, functioning as an intrinsic sensor of threats. The high sensitivity of microglial cells to the CNS microenvironment changes enables them to function as sentinels (cf. G.W. Kreutzberg, Trends Neurosci. 1996, vol. 19, pp. 312-8).

Injury to neurons rapidly changes their gene expression and stimulates nearby microglia for support. Afterwards, activated microglia may directly promote cerebral injury releasing proinflammatory cytokines that affect astrocytes, oligodendrocytes and neurons (cf. F. González-Scarano et al., Annu. Rev. Neurosci. 1999, vol. 22, pp. 219-40) and creating an autocrine loop that increases direct and indirect (i.e. microglia-produced) injury. Thus, microglial actions in the CNS present a fundamentally inflammation-like character and are central to the pathogenesis and progression of a wide variety of CNS chronic (brain, spinal cord and neuroophthalmic) disorders. Chronic inflammation involves the release of a number of mediators that may result in neuronal death and shortening of life. Aging, degenerative CNS diseases (e.g. multiple sclerosis, trauma, stroke, Alzheimer's disease, Parkinson's disease, amyotrophic lateral sclerosis, AIDS encephalitis, adrenoleukodystrophy and spinal cord injury) and CNS infectious diseases are associated with signs of chronic inflammation.

At present, treatments for CNS chronic inflammation are characterized by their nonspecific action and important undesirable side effects. Present anti-inflammatory agents are currently classified into two groups: NSAIDs (nonsteroidal anti-inflammatory drugs) and corticosteroids, mostly glucocorticoids.

NSAIDs are grouped in several chemical classes such as salicylic acid derivatives (e.g. aspirin), indole and indene acetic acids (e.g. indomethacin), heteroaryl acetic acids (e.g. diclofenac), arylpropionic acids (e.g. ibuprofen), and enolic acids (e.g. piroxicam). Their anti-inflammatory activity is mediated chiefly through inhibition of biosynthesis of prostaglandins. They do not exert a direct release inhibition of microglial pro-inflammatory cytokines such as interleukins or TNFa (tumor necrosis factor-alpha). In addition to sharing therapeutic activities, NSAIDs share several unwanted severe side effects, the most common being a propensity to induce gastric or intestinal ulceration, from a mild dyspepsia and headburn to ulceration, sometimes with fatal results. Compared to nonusers, patients who use NSAIDs on a chronic basis approximately have a three times higher relative risk for serious adverse gastrointestinal events. Other side effects of these drugs include disturbances in platelet function, prolongation of gestation or spontaneous labor and changes in renal function. Certain individuals display hypersensitivity reactions manifested by symptoms that range from vasomotor rhinitis, angioneurotic edema, urticaria and bronchial asthma to laryngeal edema and bronchostriction, hypotension and shock.

Corticosteroids, mainly glucocorticoids (e.g. prednisone), have also powerful anti-inflammatory effects through the alteration of gene expression. However, they do not address the underlying cause of the disease. In addition to their anti-inflammatory effects, they produce alterations in carbohydrate, protein and lipid metabolism, fluid and electrolyte abnormalities, hypertension, increased susceptibility to infection, myopathy, behavioral disturbances and cataracts, growth arrest, as well as the characteristic effects of steroids overdose including fat redistribution, striae, ecchymosis, acne and hirsutism. Catabolic effects on bones are also the cause of osteoporosis in Cushing's syndrome Thus, the side effects associated with chronic corticosteroid therapy are potentially life-threatening and impose a major limitation in their long-term therapeutic use.

In summary, it is highly desirable to provide new therapeutic agents for the treatment of diseases associated with signs of CNS chronic inflammation.

### SUMMARY OF THE INVENTION

Inventors have surprisingly found that human and rodent activated microglia strongly expresses a K_{ATP} channel similar to the ones known in cardiac and muscular tissues, neurons and pancreatic beta cells. This finding has lead them to provide new therapeutic agents whose action directly reduces microglial activation and thus, block its pro-inflammatory effects.

The fact that activated microglia expresses K_{ATP} channels, turns K_{ATP} channel openers (KCOs) into therapeutic targets to treat CNS inflammatory and autoimmune diseases. KCOs have been used until now against hypertension, angina pectoris, coronal heart disease, asthma, urinary incontinence and alopecia. Inventors have found that KCOs, and in particular diazoxide, result in a reduction of reactive microglial response in various CNS pathologies such as axonal injury, brain tumors, traumatic damage, neurodegeneration, spinal cord injury, infectious and autoimmune diseases.

Thus, the present invention relates to the use of a KCO, or of an isotopically species modified thereof, for the preparation of a prophylactic, therapeutic and/or diagnostic agent for CNS chronic inflammation associated with a disease or state in a mammal, including a human. The invention also provides a method of prophylaxis, therapy and/or diagnosis of a mammal, including a human, suffering from or susceptible to CNS chronic inflammation associated with a disease or state, comprising the administration of an effective amount of a KCO, or of an isotopically modified species thereof, together with appropriate amounts of acceptable diluents or carriers.

KCOs exhibit an extreme chemical diversity and comprise a number of different structural classes whose structure-activity relationship has been in part recently reviewed (cf. R. Mannhold, Med. Res. Rev. 2004, vol. 24, pp. 213-66). In a particular embodiment of the invention, the KCO is selected from the group consisting of benzopirans (e.g. cromakalim, levcromakalim, emakalim, bimakalim, celikalim, U96501, RO 31-6930, SDZ PCO 400, KC-399, KC-515, BRL 49381, JTV-506, NIP-121 and rilmakalim), cyanoguanidines (e.g. pinacidil), thioformamides (e.g. aprikalim), benzothiadiazines (e.g. diazoxide), pyridyl nitrates (e.g. nicorandil), pyrimidine sulfates (e.g. minoxidil sulfate), cyclobutenediones (e.g. WAY-151616), DHP-related compounds (e.g. ZM-244085), tertiary carbinols (e.g. ZD-6169), 6-sulfonil-chromenes, 1,2,3-triazoles, pyridothiadiazines, benzothiazines, halogenquinazolins, phenylbenzimidazoles and benzoxazine. In a more particular embodiment, the KCO is diazoxide, belonging to the benzothiadiazines class.

In a particular embodiment of the invention, CNS chronic inflammation is associated with a CNS injury and/or damage, in particular, with brain injury, spinal cord injury, global ischemia, focal ischemia, hypoxia, stroke, CNS vascular disease, neuroocular disease (e.g. inflammatory optic neuropathy and retinitis) and traumatic damage. In another embodiment, CNS chronic inflammation is associated with a CNS tumor, particularly, glioma and ganglioglioma. In another embodiment, CNS chronic inflammation is associated with a CNS degenerative disease. More particularly, the CNS degenerative disease is Alzheimer's disease, Parkinson's disease, Huntington's disease, senile dementia, taupathy (e.g. Down's syndrome and Niemann-Pick disease), amyloid angiopathy, macular degeneration, amyotrophic lateral sclerosis, multiple sclerosis, encephalopathy, adrenoleukodystrophy, Creutzfeld-Jakob's disease, prion-associated spongiform encephalopathy and any other prion-associated disease. In another embodiment, CNS chronic inflammation is associated with a CNS infectious disease, in particular viral infection (e.g. HIV encephalitis), parasitic infection (protozoal and metazoal infections), bacterial infection (e.g. purulent leptomeningitis and brain abscess), mycoplasma infection and fungal infection. In another embodiment, CNS chronic inflammation is associated with an autoimmune disease, particularly, with demyelinating diseases such as multiple sclerosis and phenylketonuria. In another embodiment, CNS chronic inflammation is associated with a nutritional, metabolic or toxic disorder, in particular with hepatic encephalopathy and lead poisoning. In another embodiment, CNS chronic inflammation is associated with brain aging.

It will be appreciated that reference to "treatment" is intended to include prophylaxis as well as the alleviation of established symptoms.

A person skilled in the art would select an appropriate administration via for KCOs, including diazoxide, such as oral, buccal, parenteral, depot or rectal administration, by inhalation or insufflation (either through the mouth or the nose). Oral and parenteral formulations are preferred.

For oral administration, KCOs may take the form of, for example, tablets or capsules prepared by conventional means with pharmaceutically acceptable excipients. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives. Preparations for oral administration may be suitably formulated to give controlled release of the active compound.

Liquid preparations for perioperative CNS surgery including brain, spinal cord and ophthalmic procedures may take the form of, for example solutions or suspensions, or they may be presented as a dry product for its direct application (e.g. powder, gel or impregnated on a solid support) or constitution with water or other suitable vehicle (e.g. sterile pyrogen-free water) before use. Such liquid preparations may be prepared by conventional means with pharmaceutically acceptable additives such as emulsifying agents (e.g. lecithin or acacia); non-aqueous vehicles (e.g. almond oil, oily esters, ethyl alcohol or fractionated vegetable oils) and preservatives (e.g. methyl or propyl-p-hydroxybenzoates or sorbic acid). The preparations may also contain buffer salts and, optionally, multiple active agents (e.g. antibiotics) in a physiological carrier, such as saline or lactated Ringer's solution, as appropriate. The solution is applied by continuous irrigation of the wound during surgical and diagnostic procedures for preventive inhibition of CNS chronic inflammation, and while avoiding undesirable side effects associated with oral, intravenous (i.v.), subcutaneous (s.c.) or intramuscular (i.m.) application of large doses of these agents.

KCOs may be formulated for parental administration by bolus injection or continuous infusion. Formulations for injection may be presented in unit dosage form (e.g. in ampoules or in multidose containers) with an added preservative. The compositions may take such forms as suspensions, solutions or emulsions in oily or aqueous vehicles, and may contain agents such as stabilizing or dispersing agents. Alternatively, the active ingredient may be in powder form for constitution with a suitable vehicle (e.g. sterile pyrogen-free water) before use.

KCOs may also be formulated as rectal compositions such as suppositories or retention enemas (e.g. containing conventional suppository bases such as cocoa butter or other glycerides).

KCOs may also be formulated as depot preparations. Such long acting formulations may be administered by implantation (e.g. s.c. or i.m.) or by intramuscular injection. Thus, for example, the compounds of the invention may be formulated with suitable polymeric or hydrophobic materials (e.g. as an emulsion in an acceptable oil) or ion exchange resins, or as sparingly soluble derivatives (e.g. as a sparingly soluble salt).

For intranasal and ocular administration and for intracerebroventricular infusion, KCOs may be formulated as solutions for administration via a suitable metered or unit dose device or alternatively as a powder mix with a suitable carrier for administration using a suitable delivery device.

Suitable doses ranges would be routinely found by the person skilled in the art. Thus, for use in conditions according to the present invention, the compounds may be used at doses appropriate for other conditions for which KCOs are known to be useful. It may be necessary to make routine variations to the dosage, depending on the age and condition of the patient, and the precise dosage will be ultimately at the discretion of the attendant physician or veterinarian. The dosage will also depend on the route of administration and the particular compound selected. A suitable dose range is for example 0.01 to 1000 mg/kg bodyweight per day, preferably from 0.1 to about 200 mg/kg, and more preferably from 0.1 mg/kg to 10 mg/kg,.

The KCOs useful in the present invention may be administered in combination with other KCOs and/or in combination with other therapeutic agents and may be formulated for administration by any convenient route in a convenient manner. Appropriate doses would be routinely found by those skilled in the art.

The invention also refers to the use of an isotopically modified KCO for the preparation of a diagnostic agent for CNS chronic inflammation. The skilled in the art would appropiately choose isotopes and techniques to detect and follow microglial reaction and thus, evolution of a patient with CNS inflammation. Functional brain imaging techniques such as positron emission tomography (PET), single-photon emission computed tomography (SPECT) and nuclear magnetic resonance (NMR) may provide an image that represents the distribution in the CNS of the microglial reaction. Once activated, microglia shows a territorially highly restricted involvement in the disease process. This confers to them diagnostic value for the accurate spatial localization of any active disease process. KCOs may be labelled for example with ¹¹C, ¹³C, ¹⁷F, ³¹P, ¹H or ¹⁷O.

Throughout the description and claims the word "comprise" and variations of the word, such as "comprising", are not intended to exclude other technical features, additives, components, or steps. The abstract of the present application is incorporated herein as reference. Additional objects, advantages and features of the invention will become apparent to those skilled in the art upon examination of the description or may be learned by practice of the invention. The following example and drawing are provided by way of illustration, and are not intended to be limiting of the present invention.

### DESCRIPTION OF THE DRAWING

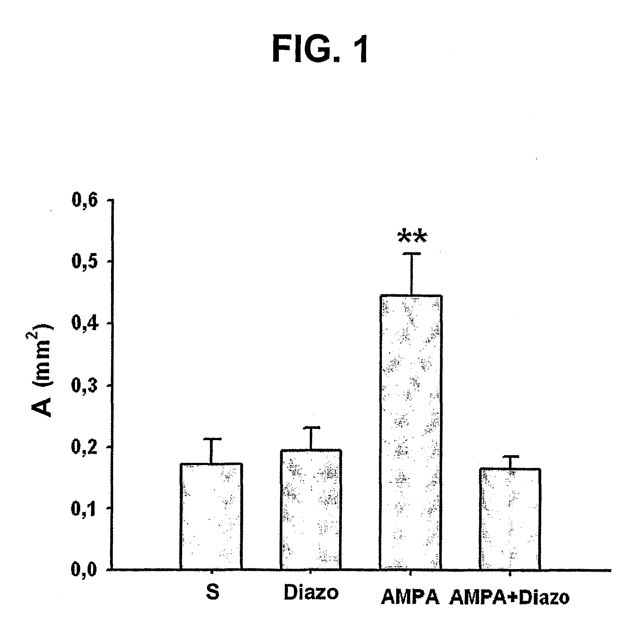

FIG. 1 shows the effect of diazoxide in reducing microglial reaction in rat hippocampal lesion. Hippocampal microgliosis area (A, in mm²) induced by 2 stereotaxic microinjection of PBS (sham, S), diazoxide (diazo), AMPA and AMPA+diazo is represented. Two asterisks represent p<0.01 different from sham (LSD post-hoc test).

### DETAILED DESCRIPTION OF A PARTICULAR EMBODIMENT

### Diazoxide reduces microglial reaction in rat hippocampal lesion

This model relies on the acute stereotaxic over-activation of rat glutamate hippocampal receptors that results in a neurodegenerative process characterized by a neuronal loss with astroglial and microglial reactions (cf. F. Bernal et al., Hippocampus 2000, vol. 10, pp. 296-304; F. Bernal et al., Exp. Neurol. 2000, vol. 161, pp. 686-95). In this neurodegenerative model rats were anaesthetized with equithesin (a mixture of chloral hydrate and pentobarbital sodium; 0.3 ml/100 g body weight, i.p.) and placed on a Kopf stereotaxic frame with the incisor bar set at -3.3 mm. Intracerebral injections aimed at the dorsal hippocampus were performed at 3.3 mm caudal to bregma, 2.2 mm lateral and 2.9 mm ventral from dura (cf. G. Paxinos et al., "The rat brain in stereotaxic coordinates", Sydney: Academic Press 1986). A volume of 0.5 *µl* was injected over a period of 5 min.

Four different groups of rats received two injections in a 2-hour interval as follows: a) sham rats (n = 4) received two injections of PBS; b) AMPA rats (α-amino-3-hydroxy-5-methylisoxazole-4-propionic acid) (n = 4) received the first injection of 5.4 mM AMPA and the second of PBS; c) diazoxide rats (n = 4) received two injections of 50 *µM* diazoxide; d) AMPA+diazo rats (n = 4) received 5.4 mM AMPA + 50 *µM* diazoxide in the first injection and 50 *µM* diazoxide in the second injection. All rats were sacrificed 24 hours after the lesion.

Rats were transcardially perfused with 300 ml of 0.1 M phosphate buffer (PB, pH 7.4) followed by 300 ml ice-cold fixative (flow rate 20 ml/min). The fixative consisted of 4% (w/v) paraformaldehyde in PB. Brains were removed, crioprotected with 15% (w/v) sucrose in PB and then, frozen with dry ice. Cryostat sections (12 µm) were obtained at the level of dorsal hippocampus (-3.3 mm to bregma). Isolectine B4 (IB4) histochemistry was performed to identify the microglial reaction (cf. C.A. Colton et al., J. Histochem. Cytochem. 1992, vol. 40, pp. 505-12).

Microgliosis area evaluation was performed on IB4 positive stained sections, which were analyzed using a computer-assisted image analysis system (OPTIMAS®, BioScan Inc., Washington, USA). IB4-stained reactive microcytes were counted at x100 magnification using an ocular grid mounted on a transmission light microscope (Axiolab, Zeiss, Göttingen, Germany). One-way ANOVA was used to compare differences between groups, followed by the LSD post-hoc test. Results were expressed as mean ± S.E.M. All analyses were performed with the computer program STATGRAPHICS (STSC Inc., Rockville, MD, USA).

The microglial reaction found in sham and diazoxide groups was similar, reaching an area of 0.17 ± 0.04 mm² and 0.19 ± 0.03 mm² respectively. In AMPA rats, a strong microgliosis was evidenced with the ameboid microcytes extended through an area of 0.44 ± 0.07 mm². In the AMPA+diazo group this microgliosis area decreased to an area of 0.16 ± 0.02 mm² (37% of the AMPA group) similar to that of sham and diazoxide groups (One-way ANOVA test result: F₃,1₂= 8.19; p = 0.0031) (cf. FIG. 1). In all four groups the density of reactive microcytes found was similar: 504 ± 82 cells/mm² for sham, 555 ± 91 cells/mm² for diazoxide, 645 ± 59 cells/mm² for AMPA and 603 ± 56 cells/mm² for AMPA+diazo.

From these results it is clear that diazoxide reduces microglial activation to sham values. A lower microglial reaction was observed in animals treated with diazoxide, in comparison with AMPA treated animals.

## Claims

1. Use of a K_{ATP} channel opener (KCO), or of an isotopically species modified thereof, for the preparation of a prophylactic, therapeutic and/or diagnostic agent for CNS chronic inflammation associated with a disease or state in a mammal, including a human.

2. The use according to claim 1, wherein the K_{ATP} channel opener is selected from the group consisting of benzopirans, cyanoguanidines, thioformamides, benzothiadiazines, pyridyl nitrates, pyrimidine sulfates, cyclobutenediones, DHP-related compounds, tertiary carbinols, 6-sulfonil-chromenes, 1,2,3-triazoles, pyridothiadiazines, benzothiazines, halogenquinazolins and phenylbenzimidazoles.

3. The use according to claim 2, wherein the K_{ATP} channel opener is selected from the group consisting of diazoxide, cromakalim, levcromakalim, emakalim, bimakalim, celikalim, U96501, RO 31-6930, SDZ PCO 400, KC-399, KC-515, BRL 49381, JTV-506, NIP-121, rilmakalim, pinacidil, aprikalim, nicorandil, minoxidil sulfate, WAY-151616, ZM-244085, ZD-6169 and benzoxazine.

4. The use according to claim 2, wherein the disease is a CNS injury and/or damage.

5. The use according to claim 4, wherein the CNS injury and/or damage is selected from the group consisting of brain injury, spinal cord injury, global ischemia, focal ischemia, hypoxia, stroke, CNS vascular disease, neuroocular disease and traumatic damage.

6. The use according to claim, 2, wherein the disease is a CNS tumor.

7. The use according to claim 6, wherein the CNS tumor is selected from the group consisting of glioma and ganglioglioma.

8. The use according to claim 2, wherein the disease is a CNS degenerative disease.

9. The use according to claim 8, wherein the CNS degenerative disease is selected from the group consisting of Alzheimer's disease, Parkinson's disease, Huntington's disease, senile dementia, taupathy, amyloid angiopathy, macular degeneration, amyotrophic lateral sclerosis, multiple sclerosis, encephalopathy, adrenoleukodystrophy, Creutzfeld-Jakob's disease, prion-associated spongiform encephalopathy and other prion-associated disease.

10. The use according to claim 2, wherein the disease is a CNS infectious disease.

11. The use according to claim 10, wherein the CNS infectious disease is selected from the group consisting of viral infection, parasitic infection, bacterial infection, mycoplasma infection and fungal infection.

12. The use according to claim 2, wherein the disease is an autoimmune disease.

13. The use according to claim 12, wherein the autoimmune disease is selected from the group consisting of multiple sclerosis and phenylketonuria.

14. The use according to claim 2, wherein the disease is a nutritional, metabolic or toxic disorder.

15. The use according to claim 14, wherein the disorder is selected from the group consisting of hepatic encephalopathy and lead poisoning.

16. The use according to claim 2, wherein the state is brain aging.

17. The use according to any of the preceding claims, wherein the K_{ATP} channel opener is diazoxide.

18. Method of prophylaxis, therapy and/or diagnosis of a mammal, including a human, suffering from or susceptible to CNS chronic inflammation associated with a disease or state, comprising the administration of an effective amount of a K_{ATP} channel opener (KCO), or of an isotopically modified species thereof, together with appropriate amounts of acceptable diluents or carriers.

19. The method according to claim 18, wherein the K_{ATP} channel opener is selected from the group consisting of benzopirans, cyanoguanidines, thioformamides, benzothiadiazines, pyridyl nitrates, pyrimidine sulfates, cyclobutenediones, DHP-related compounds, tertiary carbinols, 6-sulfonil-chromenes, 1,2,3-triazoles, pyridothiadiazines, benzothiazines, halogenquinazolins and phenylbenzimidazoles.

20. The method according to claim 19, wherein the K_{ATP} channel opener is selected from the group consisting of diazoxide, cromakalim, levcromakalim, emakalim, bimakalim, celikalim, U96501, RO 31-6930, SDZ PCO 400, KC-399, KC-515, BRL 49381, JTV-506, NIP-121, rilmakalim, pinacidil, aprikalim, nicorandil, minoxidil sulfate, WAY-151616, ZM-244085, ZD-6169 and benzoxazine.

21. The method according to claim 19, wherein the disease is a CNS injury and/or damage.

22. The method according to claim 21, wherein the CNS injury and/or damage is selected from the group consisting of brain injury, spinal cord injury, global ischemia, focal ischemia, hypoxia, stroke, CNS vascular disease, neuroocular disease and traumatic damage.

23. The method according to claim 19, wherein the disease is a CNS tumor.

24. The method according to claim 23, wherein the CNS tumor is selected from the group consisting of glioma and ganglioglioma.

25. The method according to claim 19, wherein the disease is a CNS degenerative disease.

26. The method according to claim 25, wherein the CNS degenerative disease is selected from the group consisting of Alzheimer's disease, Parkinson's disease, Huntington's disease, senile dementia, taupathy, amyloid angiopathy, macular degeneration, amyotrophic lateral sclerosis, multiple sclerosis, encephalopathy, adrenoleukodystrophy, Creutzfeld-Jakob's disease, prion-associated spongiform encephalopathy and other prion-associated disease.

27. The method according to claim 19, wherein the disease is a CNS infectious disease.

28. The method according to claim 27, wherein the CNS infectious disease is selected from the group consisting of viral infection, parasitic infection, bacterial infection, mycoplasma infection and fungal infection.

29. The method according to claim 19, wherein the disease is an autoimmune disease.

30. The method according to claim 29, wherein the autoimmune disease is selected from the group consisting of multiple sclerosis and phenylketonuria.

31. The method according to claim 19, wherein the disease is a nutritional, metabolic or toxic disorder.

32. The method according to claim 31, wherein the disorder is selected from the group consisting of hepatic encephalopathy and lead poisoning.

33. The method according to claim 19, wherein the state is brain aging.

34. The method according to any of the claims 18-33, wherein the K_{ATP} channel opener is diazoxide.
